# EUROPEAN PATENT SPECIFICATION

(11) **EP 0 353 910 B1**
(45) Date of publication and mention of the grant of the patent: **20.09.1995**
(21) Application number: 89307374.2
(22) Date of filing: 20.07.1989
(51) Int. Cl.: C12P 21/00, C12N 5/00, A61K 38/21

(54) **Human interferon-gamma, process for preparing said human interferon-gamma, and its use**
Menschliches Gamma-Interferon, Verfahren zu seiner Herstellung und seine Verwendung
Gamma-interféron humain, son procédé de préparation et son utilisation

(30) Priority: 23.07.1988 JP 184069/88
(43) Date of publication of application: 07.02.1990
(73) Proprietor: KABUSHIKI KAISHA HAYASHIBARA SEIBUTSU KAGAKU KENKYUJO, Okayama-shi Okayama (JP)
(72) Inventor: Kurimoto, Masashi, Okayama-shi Okayama (JP); Mitsuhashi, Masakazu, Okayama-shi Okayama (JP)
(74) Representative: Pendlebury, Anthony

(56) References cited:
- EP-A- 0 107 498
- EP-A- 0 128 009
- EP-A- 0 254 593
- WO-A-83/01899
- EUROPEAN JOURNAL OF BIOCHEMISTRY, vol. 156, 1986, pages 651-654, FEBS; J.H.G.M. MUTSAERS et al.: "Structural studies of the carbohydrate chains of human gamma-interferon"
- LOIS B. EPSTEIN: "Interferon", vol. 2: "Interferons and immune system", J. Vilcek and E. De Maeyer Eds. 1984, Elsevier Science Publishers B.V., chapter 12, pages 185-189

## Description

The present invention relates to an HuIFN-gamma, a process for preparing said HuIFN-gamma, and its use (hereinafter abbreviated as "HuIFN-gamma"), more particularly, to an HuIFN-gamma having a novel structure, a process for preparing said HuIFN-gamma, and an agent containing said HuIFN-gamma as an effective component, which is directed to prevention or treatment of HuIFN-gamma-susceptive diseases.

It has been known that human interferons (HuIFNs) include HuIFN-alpha (or leukocyte HuIFN), HuIFN-beta (or fibroblast HuIFN), and HuIFN-gamma (or immune HuIFN or type II HuIFN): For HuIFN-alpha and HuIFN-beta, some processes using lymphoblast- and fibroblast-cells respectively have been developed, and, recently, pharmaceuticals containing these HuIFNs have been commercialized.

While many processes have been proposed for preparing an HuIFN-gamma, none of them has been practised on an industrial-scale.

In general, the process which enables a mass production of HuIFN-gamma, and has been deemed most realizable is that using a gene recombinant technique wherein E. coli is utilized (see Japanese Patent Laid-Open Nos.201,995/83 and 202,899/85).

However, an HuIFN-gamma obtained with the process is far from a natural HuIFN-gamma because the former HuIFN-gamma consists of a polypeptide chain structure, while the latter HuIFN-gamma comprises carbohydrate chain structure. For this reason, a gene coding an HuIFN-gamma has been introduced into a Chinese hamster ovary cell (CHO cell), which is then allowed to release an HuIFN-gamma having a carbohydrate chain structure, and recovering the resultant HuIFN-gamma (Japanese Patent Laid-Open No.289,600/87).

European Journal of Biochemistry, Vol.156, pp.651-654 (1986) details the carbohydrate chain structure as follows: 
[NeuAcα(2-3)]0-1 Galβ(1-4)GlcNAcβ(1-2)Manα(1-3)
Manβ(1-4)GlcNAcβ(1-4)GlcNAc-ol
[NeuAcα(2-3)]0-1 Galβ(1-4)GlcNAcβ(1-2)Manα(1-6)
[Fucα(1-6)]0-1
This report, however, only teaches the carbohydrate chain structure of an HuIFN-gamma derived from a CHO cell.

A partial carbohydrate chain structure of an HuIFN-gamma derived from a human cell is reported in Nikkei Biotechnology, p.5, (22. 9. 1986): 
(NeuAc)-Gal-GlcNAc-Man
>Man-GlcNAc-GlcNAc-Asn
(NeuAc)-Gal-GlcNAc-Man
This report, however, disclosed no coupling mode in the carbohydrate chain structure of these HuIFN-gamma, and whether the carbohydrate chain structures are identical or not is not clear.

In order to treat or prevent human diseases, the use of an HuIFN-gamma, essentially derived from a human cell, is safe and desirable because it causes no side effect such as antigen-antibody reaction.

The structure of a natural HuIFN-gamma has now been deduced by exposing a human cell to the action of an inducer toproduce a natural HuIFN-gamma; highly purifying and recovering the resultant HuIFN-gamma; and analyzing its polypeptide chain structures and carbohydrate chain structures. As a result, it has been found that a natural HuIFN-gamma, which may be produced by exposing a human cell to the action of aninducer, is a glycoprotein having a novel structure.

The present invention therefore provides a human interferon-gamma which consists essentially of a polypeptide chain having a sequence as shown in any one of the following Formulae:- 
Formula I:
1                                                    10
pyroGlu Asp Pro Tyr Val Lys Glu Ala Glu Asn Leu Lys Lys Tyr Phe
                    20                                          30
Asn Ala Gly His Ser Asp Val Ala Asp Asn Gly Thr Leu Phe Leu
                                        40
Gly Ile Leu Lys Asn Trp Lys Glu Glu Ser Asp Arg Lys Ile Met
 
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu
130
Ser Pro Ala Ala Lys Thr Gly Lys Arg Lys Arg Ser Gln Met Leu

Phe Agr Gly ;

Formula II:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys Arg;
 
Formula III:
1                                                    10
pyroGlu Asp Pro Tyr Val Lys Glu Ala Glu Asn Leu Lys Lys Tyr Phe
                    20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                    80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                    110                                                   120
Gln Arg Lys Ala Ile His Glu Leu Ile Gln Val Met Ala Glu Leu

Ser Pro Ala Ala Lys Thr Gly Lys;
Formula IV:
1                                                    10
pyroGlu Asp Pro Tyr Val Lys Glu Ala Glu Asn Leu Lys Lys Tyr Phe
                    20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                    80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                    110                                                   120
Gln Arg Lys Ala Ile His Glu Leu Ile Gln Val Met Ala Glu Leu

Ser Pro Ala Ala Lys Thr Gly;or
 
Formula V:
1                                                    10
pyroGlu Asp Pro Tyr Val Lys Glu Ala Glu Asn Leu Lys Lys Tyr Phe
                    20                                          30
Asn Ala Gly His Ser Asp Val Ala Asp Asn Gly Thr Leu Phe Leu
                                        40
Gly Ile Leu Lys Asn Trp Lys Glu Glu Ser Asp Arg Lys Ile Met
                    50                                          60
Gln Ser Gln Ile Val Ser Phe Tyr Phe Lys Leu Phe Lys Asn Phe
                                        70
Lys Asp Asp Gln Ser Ile Gln Lys Ser Val Glu Thr Ile Lys Glu
                    80                                          90
Asp Met Asn Val Lys Phe Phe Asn Ser Asn Lys Lys Lys Arg Asp
                                        100
Asp Phe Glu Lys Leu Thr Asn Tyr Ser Val Thr Asp Leu Asn Val
                    110                                         120
Gln Arg Lys Ala Ile His Glu Leu Ile Gln Val Met Ala Glu Leu

Ser Pro Ala Ala Lys Thr ;
(wherein the abbreviations are commonly used for L-amino acids in the art) ;
wherein either or both of asparagines which locate at the twenty-fifth and ninety-seventh positions with respect to the NH₂ terminus of the polypeptide chain is coupled to a carbohydrate chain having a structure as shown in any one of the following Formulae:- 
Formula VI:

NeuAcα(2→6)Galβ(1→4)GlcNAcβ(1→2)Manα 1                    Fucα 1
                                                                                    ↓                                  ↓
                                                                                    6                                  6
                                                                                  Manβ(1→4)GlcNAcβ(1→4)GlcNAc→   ;
                                                                                    3
                                                                                    ↑
NeuAcα(2→6)Galβ(1→4)GlcNAcβ(1→2)Manα 1
 
Formula VIII:

NeuAcα(2→3)Galβ(1→4)GlcNAcβ(1→2)Manα 1                    Fucα 1
                                                                                    ↓                                    ↓
                                                                                    6                                    6
                                                                                    Manβ(1→4)GlcNAcβ(1→4)GlcNAc→    ;
                                                                                    3
                                                                                    ↑
NeuAcα(2→6)Galβ(1→4)GlcNAcβ(1→2)Manα 1

Formula X:

                    Galβ(1→4)GlcNAcβ(1→2)Manα 1                    Fucα 1
                                                                                    ↓                                    ↓
                                                                                    6                                    6
                                                                                    Manβ(1→4)GlcNAcβ(1→4)GlcNAc→
                                                                                    3
                                                                                    ↑
NeuAcα(2→6)Galβ(1→4)GlcNAcβ(1→2)Manα 1                                                    ;or

Formula XI:

NeuAcα(2→3)Galβ(1→4)GlcNAcβ(1→2)Manα 1                    Fucα 1
                                                                                    ↓                                    ↓
                                                                                    6                                    6
                                                                                    Manβ(1→4)GlcNAcβ(1→4)GlcNAc→    ,
                                                                                    3
                                                                                    ↑
                    Galβ(1→4)GlcNAcβ(1→2)Manα 1
                                        3
                                        ↑
                              Fucα 1
(wherein NeuAc, GlcNAc, Gal, Man and Fuc mean N-acetylneuraminic acid, N-acetyl-D-glucosamine, D-galactose, D-mannose and L-fucose respectively), as well as establishing a use of the HuIFN-gamma.

Since the present invention revealed an HuIFN-gamma with a novel structure, any process to prepare the HuIFN-gamma can be freely used.

A natural HuIFN-gamma may be prepared by exposing a human cell to the action of an inducer to induce the HuIFN-gamma, which is then purified and recovered.

Furthermore, the present HuIFN-gamma can be advantageously produced in an increased yield with no exposure to an inducer by introducing a HuIFN-gamma gene into a human cell, and by allowing a human cell capable of producing an HuIFN-gamma to fuse with another human cell.

The human cell may be obtained by
implanting a human cell capable of producing said human interferon-gamma in a non-human warm-blooded animal; and
allowing said human cell to proliferate while allowing it to receive the body fluid From the non-human warm-blooded animal.

If necessary, the present HuIFN-gamma can be advantageously prepared by employing a method wherein an HuIFN-gamma polypeptide chain which is prepared by a recombinant microorganism such as recombinant E. coli, or, by organic and chemical synthesis, or biochemical synthesis, is subjected, for example, to the action of a glycosyl transferase to transfer a carbohydrate chain to the polypeptide chain in a biochemical manner.

The human cell usable in the above is an established human cell, for example, BALL-1 cell (JCRB 0071), TALL-l cell (JCRB 0086) or MOLT-3 cell (ATCC CRL 1552) disclosed in Japanese Patent Publication No.1,296/88, or a human myelomonocyte, for example, HBL-38 cell, HL-60 cell (ATCC CCL 240), KG-1 cell (ATCC CCL 246) or ML-1 cell disclosed in Japanese Patent Laid-Open No.152,993/88; or, if necessary, a leukocyte cell such as buffy coat, prepared from human peripheral blood, may be used.

A preparation of an HuIFN-gamma using these cells, is generally carried out in a manner that the cells are suspended in a nutrient culture medium, kept at about 20-40°C, to give a cell density of about 10⁵×10⁸ cells/ml, and exposed to the action of an inducer.

Such inducer should be capable of inducing an HuIFN-gamma production, for example, mitogens such as phytohemagglutinin, concanavalin A, pokeweed mitogen, lipopolysaccharide, lipid A, endotoxin, polysaccharide, or bacterium.

Furthermore, antigens act on sensitized cells to induce HuIFN-gamma production.

The above HuIFN-gamma inducers are usually used at a concentration of from about 0.001 microgram/ml to 10 mg/ml. They can be used together with HuIFN-alpha inducers, for example, virus, nucleic acid and polynucleotide, if necessary.

The HuIFN-gamma thus obtained can be easily isolated, purified and recovered by conventional purification and isolation methods, for example, salting-out, dialysis, filtration, centrifugation, concentration and lyophilization.

When further purification is required, one or more conventional procedures, for example, adsorption and desorption with ion-exchange, gel filtration, affinity chromatography, isoelectric point fractionation, high-performance liquid chromatography and electrophoresis, are used in combination. In particular, chromatography using a monoclonal antibody leads to an HuIFN-gamma of the highest purity.

The HuIFN-gamma thus obtained can be advantageously used in prophylactic or therapeutic agents for HuIFN-gamma-susceptive diseases.

The wording "HuIFN-gamma-susceptive diseases" means those which can be prevented and/or treated with an HuIFN-gamma, for example, viral diseases such as an epidemic conjunctivitis, herpetic keratitis, influenza, rubella, serum hepatitis, and acquired immune deficiency syndrome (AIDS); nonviral diseases; malignant tumors such as colon carcinoma, lung carcinoma, liver carcinoma and osteosarcoma; and immunopathies such as atopic allergy, myasthenia gravis, collagenosis, pernicious anemia, articular rheumatism, and systemic lupus erythematosus.

The form of the agent according to the present invention can be freely chosen in accordance with its final uses, for example, a nebula, collyrium, a collutory, a liquid medicine such as an injection, a paste medicine such as an ointment, and a solid medicine such as a powder, a granule or a tablet.

Into the agent is incorporated the HuIFN-gamma in the range of 1-10,000,000 units/g, if necessary, together with one or more lymphokines such as IFN-alpha, IFN-beta, tumor necrosis factor (hereinafter abbreviated as "TNF"), lymphotoxin, interleukin 2, and B-cell differentiating factor, or natural or synthetic chemotherapeutic(s) so that the efficacy of the agent is advantageously augmented.

If necessary, the HuIFN-gamma can be freely used in combination with an adjuvant, a filler and/or a stabilizer. The agent thus obtained is advantageously used, for example, in an antiviral agent, an antioncotic, an enhancer for antioncotic chemotherapeutics, a depressant for matastasis of malignant tumors, a suppressant for palindromia, an immunoregulator, and an agent for immunopathies.

The activity of IFNs with a high human species-specificity was determined by the plaque reduction method, using FL cell, derived from human amnion, described in Protein, Nucleic Acid and Enzyme, Vol.20, No.6, pp.616-643 (1975).

When an HuIFN-gamma is in a mixture with HuIFN-alpha and HuIFN-beta, the mixture was pretreated with anti-HuIFN-alpha and anti-HuIFN-beta antibodies to neutralize both HuIFN-alpha and HuIFN-beta, and the resultant was determined for its HuIFN-gamma activity.

The present invention is illustrated in more detail by the following Examples.

### Example A-1

### HuIFN-gamma

### Example A-1-(1)

### Preparation of high-purity HuIFN-gamma

Newborn hamsters were injected with an antiserum, prepared from rabbit in conventional manner, to weaken possible immunoreaction, implanted subcutaneously with KG-1 cell (ATCC CCL246), and fed in usual manner for 3 weeks. The tumor masses, formed subcutaneously in the hamsters, about 15 g each, were extracted and disaggregated by suspending them in a physiological saline containing collagenase. The resultant cells were recovered, washed with RPMI 1640 medium, and resuspended in a fresh preparation of the same culture medium to give a cell density of about 2x10⁶ cells/ml. The cell suspension was added with about 5 micrograms/ml lipopolysaccharide, and incubated at 37°C for 2 days to induce HuIFN-gamma production. The resultant culture was centrifugally separated to obtain a supernatant containing about 45,000 units/ml supernatant of HuIFN-gamma, or about 35,000,000 units of HuIFN-gamma per hamster. The supernatant was concentrated with a membrane filter, sterilely filtered and subjected to chromatography using a column of a monoclonal anti-HuIFN-gamma antibody, a trade name of γ-Sepharose, Celltech, Ltd., Berkshire, UK. The resultant solution was concentrated, and subjected to gel chromatography to obtain a fraction exhibiting HuIFN-gamma activity. Then, an HuIFN-gamma solution was recovered in the yield of an about 80% with respect to HuIFN-gamma activity.

The product was a high-purity HuIFN-gamma having a specific activity of about 2×10⁷ units/mg protein.

### Example A-1-(2)

### Molecular weight of HuIFN-gamma

After feeding a high-purity HuIFN-gamma, prepared by the method in Example A-1-(1), to SDS-polyacrylamide gel electrophoresis in accordance with the method described by U. K. Laemmli, in Nature, Vol.227, p.680-685 (1970), several bands were observed around the molecular weight of about 24,000, 20,000 and 16,000 daltons. The amounts of protein in the bands were about 7:2:1 based on their molecular ratios respectively. HuIFN-gamma activity was detected in each band. Furthermore, the presence of carbohydrate chains was checked by periodic acid Shiff base stain in accordance with the method described by R. A. Kapitany and E. J. Zebrowski, in Analytical Biochemistry, Vol.56, p.361-369 (1973), and the bands having a molecular weight of about 24,000 and about 20,000 daltons were determined positive.

### Example A-1-(3)

### Amino acid sequence of HuIFN-gamma

The amino acid sequence of a high-purity HuIFN-gamma, prepared by the method in Example A-1-(1), was determined in accordance with the method described by Ernst Rinderknecht et al., in The Journal of Biological Chemistry, Vol.259, No.11, pp.6790-6797 (1984).

The HuIFN-gamma was digested with trypsin or V8 protease, a product of Sigma Chemical Company, St., Louis, Mo., USA, and the resultant peptide fragments were fractionated with high-performance liquid chromatography. Each fragment was fed to "Model 470A", a gas-phase protein sequencer, commercialized by Applied Biosystems, Inc., CA, USA, and then analyzed with high-performance liquid chromatography to determine the amino acid sequence of HuIFN-gamma. Carbohydrate chains coupled to peptide fragments were first cut with glycopeptidase A, a product of Seikagaku Kogyo Kabushiki Kaisha, Tokyo, Japan, then fed to a gas-phase protein sequencer.

The evidence confirmed that the present HuIFN-gamma has a polypeptide chain shown by the following formula as the main structure: 
Formula I:
1                                                    10
pyroGlu Asp Pro Tyr Val Lys Glu Ala Glu Asn Leu Lys Lys Tyr Phe
                    20                                          30
Asn Ala Gly His Ser Asp Val Ala Asp Asn Gly Thr Leu Phe Leu
                                        40
Gly Ile Leu Lys Asn Trp Lys Glu Glu Ser Asp Arg Lys Ile Met
 
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu
130
Ser Pro Ala Ala Lys Thr Gly Lys Arg Lys Arg Ser Gln Met Leu
Phe Agr Gly

Formula II:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu
Ser Pro Ala Ala Lys Thr Gly Lys Arg
 
Formula III:

1                                                    10
pyroGlu Asp Pro Tyr Val Lys Glu Ala Glu Asn Leu Lys Lys Tyr Phe
                        20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                        50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                        80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                        110                                                   120
Gln Arg Lys Ala Ile His Glu Leu Ile Gln Val Met Ala Glu Leu

Ser Pro Ala Ala Lys Thr Gly Lys

Formula IV:

1                                                    10
pyroGlu Asp Pro Tyr Val Lys Glu Ala Glu Asn Leu Lys Lys Tyr Phe
                        20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                        50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                        80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
 
110                                                    120
Gln Arg Lys Ala Ile His Glu Leu Ile Gln Val Met Ala Glu Leu

Ser Pro Ala Ala Lys Thr Gly, or

Formula V:

1                                          10
pyroGlu Asp Pro Tyr Val Lys Glu Ala Glu Asn Leu Lys Lys Tyr Phe

                    20                                          30
Asn Ala Gly His Ser Asp Val Ala Asp Asn Gly Thr Leu Phe Leu

                                          40
Gly Ile Leu Lys Asn Trp Lys Glu Glu Ser Asp Arg Lys Ile Met

                    50                                          60
Gln Ser Gln Ile Val Ser Phe Tyr Phe Lys Leu Phe Lys Asn Phe

                                          70
Lys Asp Asp Gln Ser Ile Gln Lys Ser Val Glu Thr Ile Lys Glu

                    80                                          90
Asp Met Asn Val Lys Phe Phe Asn Ser Asn Lys Lys Lys Arg Asp

                                          100
Asp Phe Glu Lys Leu Thr Asn Tyr Ser Val Thr Asp Leu Asn Val

                    110                                         120
Gln Arg Lys Ala Ile His Glu Leu Ile Gln Val Met Ala Glu Leu

Ser Pro Ala Ala Lys Thr
In the above formula, a carbohydrate chain in the form of glycosylamine type of chain is coupled to either or both asparagines which are located at the twenty-fifth and ninety-seventh positions from the NH₂ terminus of the polypeptide chain.

In other words, the polypeptide chain shown by Formula II lacks 9 amino acids from the COOH terminus of the polypeptide chain shown by Formula I, and, similarly, the polypeptide chains shown by Formulas III, IV and V respectively lack 10, 11 and 12 amino acids from the COOH terminus of the polypeptide chain shown by Formula I. Specifically, it was also confirmed that the content of the polypeptide chain shown by Formula IV is relatively high, or about 50-80% based on molecular ratios of these polypeptide chains.

### Example A-1-(4)

### Carbohydrate chain structure of HuIFN-gamma

The carbohydrate chain structure of a high-purity HuIFN-gamma, prepared by the method in Example A-1-(1), was studied.

### Example A-1-(4)-(a)

### Non-ionic saccharide and amino sugar composing carbohydrate chain of HuIFN-gamma

In accordance with the method described in European Journal of Biochemistry, Vol.156, pp.651-654 (1986), an HuIFN-gamma was subjected to methanolysis, and the resultant was trimethylcylylated, and fed to gas-chromatography, followed by determining a non-ionic saccharide in the HuIFN-gamma. Furthermore, an HuIFN-gamma was first hydrolyzed with methanesulfonic acid in accordance with the method described by R. J. Simpson et al., in The Journal of Biological Chemistry, Vol.251, No.7, pp.1936-1940 (1976), then fed to an amino acid analyzer to determine the amino sugar of HuIFN-gamma in accordance with the method described by A. M. Bella et al., in Journal of Chromatography, Vol.51, pp.314-315 (1970).

The results confirmed that both non-ionic saccharide and amino sugar consisted of D-mannose, D-galactose, L-fucose and N-acetyl-D-glucosamine in a molecular ratio of 3.00:2.16:0.82:3.94.

### Example A-1-(4)-(b)

### Arrangement and coupling mode of carbohydrate chain in HuIFN-gamma

In accordance with the method described by S. Hase et al., in The Journal of Biochemistry, Vol.95, pp.197-203 (1984), an HuIFN-gamma was subjected to hydrazinolysis and pyridylamination, and the resultant was then fed to gel chromatography to remove excessive amount of reagents to obtain a pyridylamino sugar chain, followed by determining an arrangement and a coupling mode of a carbohydrate chain in the HuIFN-gamma.

Anion exchange chromatography revealed that the pyridylamino sugar contained a monosialylated oligosaccharide and a disialylated oligosaccharide.

Furthermore, the pyridylamino sugar was digested with neuraminidase to obtain a disialylated pyridylamino sugar chain which was then fractionated according to molecular sizes with high-performance liquid chromatography. Each fraction was subjected to successive degradation using exo-glycosidase to determine the arrangement of the carbohydrate chain of HuIFN-gamma.

Furthermore, the pyridylamino sugar was fractionated with reverse-phase column chromatography, and the resultant fractions were respectively subjected to 500-MHz ¹H-NMR spectroscopy to determine their coupling mode.

The results confirmed that the aforementioned polypeptide chain of the HuIFN-gamma was coupled to a carbohydrate chain shown by the following formula in the form of glycosylamine type of chain: 
Formula VI:

NeuAcα(2→6)Galβ(1→4)GlcNAcβ(1→2)Manα 1
↓
6
Fucα 1
↓
6
Manβ(1→4)GlcNAcβ(1→4)GlcNAc→
3
↑
NeuAcα(2→6)Galβ(1→4)GlcNAcβ(1→2)Manα 1

Formula VII:

NeuAcα(2→6)Galβ(1→4)GlcNAcβ(1→2)Manα 1
↓
6
Manβ(1→4)GlcNAcβ(1→4)GlcNAc→
3
↑
NeuAcα(2→6)Galβ(1→4)GlcNAcβ(1→2)Manα 1

Formula VIII:

NeuAcα(2→3)Galβ(1→4)GlcNAcβ(1→2)Manα 1
↓
6
Fucα 1
↓
6
Manβ(1→4)GlcNAcβ(1→4)GlcNAc→
3
↑
NeuAcα(2→6)Galβ(1→4)GlcNAcβ(1→2)Manα 1

Formual IX:

NeuAcα(2→3)Galβ(1→4)GlcNAcβ(1→2)Manα 1
↓
6
Manβ(1→4)GlcNAcβ(1→4)GlcNAc→
3
↑
NeuAcα(2→6)Galβ(1→4)GlcNAcβ(1→2)Manα 1
 
Formual X:

Galβ(1→4)GlcNAcβ(1→2)Manα 1
↓
6
Fucα 1
↓
6
Manβ(1→4)GlcNAcβ(1→4)GlcNAc→
3
↑
NeuAcα(2→6)Galβ(1→4)GlcNAcβ(1→2)Manα 1
,or

Formula XI:

NeuAcα(2→3)Galβ(1→4)GlcNAcβ(1→2)Manα 1
↓
6
Fucα 1
↓
6
Manβ(1→4)GlcNAcβ(1→4)GlcNAc→
3
↑
Galβ(1→4)GlcNAcβ(1→2)Manα 1
3
↑
Fucα 1
These structures of carbohydrate chains of HuIFN-gamma, which had not been found in IFN-alpha and IFN-beta, as well as in the HuIFN-gamma derived from CHO cell, were revealed by the present invention.

Furthermore, the content of these novel carbohydrate chains was relatively high, i.e., about 50-80% based on a molecular ratio.

It was found that the present HuIFN-gamma also contained a small amount of carbohydrate chain which had been found in a CHO cell-derived HuIFN-gamma.

The results confirmed that the present HuIFN-gamma having a molecular weight of about 24,000 daltons has carbohydrate chains coupled to both asparagines which locate at the twenty-fifth and ninety-seventh positions with respect to the NH₂ terminus of the polypeptide chain, and the present HuIFN-gamma having a molecular weight of about 20,000 daltons has a carbohydrate chain which is coupled to one of the asparagines.

In conclusion, the present HuIFN-gamma derived from a human cell is a novel HuIFN-gamma having a polypeptide chain shown by the following formula as the main structure: 
Formula I:
1                                                    10
pyroGlu Asp Pro Tyr Val Lys Glu Ala Glu Asn Leu Lys Lys Tyr Phe
                    20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                    80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                    110                                                   120
Gln Arg Lys Ala Ile His Glu Leu Ile Gln Val Met Ala Glu Leu
                                                    130
Ser Pro Ala Ala Lys Thr Gly Lys Arg Lys Arg Ser Gln Met Leu

Phe Agr Gly

Formula II:
1                                                    10
pyroGlu Asp Pro Tyr Val Lys Glu Ala Glu Asn Leu Lys Lys Tyr Phe
                    20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
 
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 12[?]
Gln Arg Lys Ala Ile His Glu Leu Ile Gln Val Met Ala Glu Leu

Ser Pro Ala Ala Lys Thr Gly Lys Arg

Formula III:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met

50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys

Formula IV:
1 10
pyroGlu Asp Pro Tyr Val Lys Glu Ala Glu Asn Leu Lys Lys Tyr Phe
 
20                                                    30
Asn Ala Gly His Ser Asp Val Ala Asp Asn Gly Thr Leu Phe Leu
                                        40
Gly Ile Leu Lys Asn Trp Lys Glu Glu Ser Asp Arg Lys Ile Met
                    50                                      60
Gln Ser Gln Ile Val Ser Phe Tyr Phe Lys Leu Phe Lys Asn Phe
                                        70
Lys Asp Asp Gln Ser Ile Gln Lys Ser Val Glu Thr Ile Lys Glu
                    80                                      90
Asp Met Asn Val Lys Phe Phe Asn Ser Asn Lys Lys Lys Arg Asp
                                        100
Asp Phe Glu Lys Leu Thr Asn Tyr Ser Val Thr Asp Leu Asn Val
                    110                                     120
Gln Arg Lys Ala Ile His Glu Leu Ile Gln Val Met Ala Glu Leu

Ser Pro Ala Ala Lys Thr Gly ,or

Formula V:

1                                       10
pyroGlu Asp Pro Tyr Val Lys Glu Ala Glu Asn Leu Lys Lys Tyr Phe
                    20                                      30
Asn Ala Gly His Ser Asp Val Ala Asp Asn Gly Thr Leu Phe Leu
                                        40
Gly Ile Leu Lys Asn Trp Lys Glu Glu Ser Asp Arg Lys Ile Met
                    50                                      60
Gln Ser Gln Ile Val Ser Phe Tyr Phe Lys Leu Phe Lys Asn Phe
                                        70
Lys Asp Asp Gln Ser Ile Gln Lys Ser Val Glu Thr Ile Lys Glu
                    80                                      90
Asp Met Asn Val Lys Phe Phe Asn Ser Asn Lys Lys Lys Arg Asp
                                        100
Asp Phe Glu Lys Leu Thr Asn Tyr Ser Val Thr Asp Leu Asn Val
                    110                                     120
Gln Arg Lys Ala Ile His Glu Leu Ile Gln Val Met Ala Glu Leu

Ser Pro Ala Ala Lys Thr
(wherein the abbreviations are commonly used for L-amino acids in the art)
wherein either or both of asparagines which locate at the twenty-fifth and ninety-seventh positions with respect to the NH₂ terminus of the polypeptide chain is coupled to a carbohydrate chain shown by 
Formula VI:
NeuAcα(2→6)Galβ(1→4)GlcNAcβ(1→2)Manα1                    Fucα1
↓                                                                                                    ↓
6                                                                                                    6
Manβ(1→4)GlcNAcβ(1→4)GlcNAc→
3
↑
NeuAcα(2→6)Galβ(1→4)GlcNAcβ(1→2)Manα1

Formula VII:
NeuAcα(2→6)Galβ(1→4)GlcNAcβ(1→2)Manα1
↓
6
Manβ(1→4)GlcNAcβ(1→4)GlcNAc→
3
↑
NeuAcα(2→6)Galβ(1→4)GlcNAcβ(1→2)Manα1

Formula VIII:
NeuAcα(2→3)Galβ(1→4)GlcNAcβ(1→2)Manα1                    Fucα1
↓                                                                                                    ↓
6                                                                                                    6
Manβ(1→4)GlcNAcβ(1→4)GlcNAc→
3
↑
NeuAcα(2→6)Galβ(1→4)GlcNAcβ(1→2)Manα1

Formula IX:
NeuAcα(2→3)Galβ(1→4)GlcNAcβ(1→2)Manα1
↓
6
Manβ(1→4)GlcNAcβ(1→4)GlcNAc→
3
↑
NeuAcα(2→6)Galβ(1→4)GlcNAcβ(1→2)Manα1
 
Formula X:

Galβ(1→4)GlcNAcβ(1→2)Manα1                    Fucα1
                                    ↓                              ↓
                                    6                              6
                                    Manβ(1→4)GlcNAcβ(1→4)GlcNAc→
                                    3
                                    ↑
NeuAcα(2→6)Galβ(1→4)GlcNAcβ(1→2)Manα1                                        ,or

Formula XI:

NeuAcα(2→3)Galβ(1→4)GlcNAcβ(1→2)Manα1                    Fucα1
                                    ↓                              ↓
                                    6.                             6
                                    Manβ(1→4)GlcNAcβ(1→4)GlcNAc→
                                    3
                                    ↑
Galβ(1→4)GlcNAcβ(1→2)Manα1
                3
                ↑
Fucα1
The product can be advantageously used as an effective component in therapeutic or prophylactic agents for viral diseases and malignant tumors, as well as a material for preparing HuIFN-gamma derivatives.

### Example A-2

### HuIFN-gamma

In accordance with the method in Example A-1-(1), newborn hamsters were implanted with HBL-38 cell, and fed in usual manner for 4 weeks. The tumor masses, formed subcutaneously in the hamsters, about 20 g each, were extracted, and disaggregated by suspending them in physiological saline containing collagenase, followed by recoverying the resultant cells.

The cells were washed with Eagle's minimum essential medium, and diluted with a fresh preparation of the same culture medium kept at 37°C, a cell density of about 2×10⁶ cells/ml. The cell suspension was then added with 200 micrograms/ml phytohemagglutinin and 5 micrograms/ml lipid A, and incubated at 37°C for 2 days to induce HuIFN-gamma production. The resultant culture was centrifugally separated to obtain a supernatant having about 93,000 units/ml supernatant of HuIFN-gamma. About 183,000,000 units of HuIFN-gamma per hamster was obtained.

In accordance with the method in Example A-1-(1), the supernatant was purified to obtain an HuIFN-gamma solution in the yield of an about 80% with respect to HuIFN-gamma activity.

The product was a high-purity HuIFN-gamma having a specific activity of about 2×10⁷ units/mg protein.

Studies on molecular weight, amino acid sequence and carbohydrate chain structure of the high-purity HuIFN-gamma in accordance with the method in Examples A-1-(2), A-1-(3) and A-1-(4), showed that the HuIFN-gamma was a novel HuIFN-gamma which had the same structure as the HuIFN-gamma in Example A-1.

Similarly as the product in Example A-1, the product can be advantageously used as an effective component in prophylactic or therapeutic agents for diseases, as well as a material for preparing HuIFN-gamma derivatives.

### Example A-3

### HuIFN-gamma

A buffy coat, prepared from human peripheral blood, was suspended in Eagle's minimum essential medium supplemented with 10 v/v % of fetal calf serum to give a cell density of 2.5×10⁶ cells/ml. To the cell suspension was added about 10 micrograms/ml phytohemagglutinin, and incubated at 37°C for 3 days to induce HuIFN-gamma production. The resultant culture medium was centrifugally separated to obtain a supernatant having about 1,000 units/ml supernatant of HuIFN-gamma.

The supernatant was purified in accordance with the method in Example A-1-(1) to obtain an HuIFN-gamma solution in the yield of an about 75% with respect to HuIFN-gamma activity.

The product was a high-purity HuIFN-gamma having a specific activity of about 2×10⁷ units/mg protein.

Studies on molecular weight, amino acid sequence and carbohydrate chain structure of the high-purity HuIFN-gamma in accordance with the method in Examples A-1-(2), A-1-(3) and A-1-(4), showed that the HuIFN-gamma was a novel HuIFN-gamma which had the same structure as the HuIFN-gamma in Example A-1.

Similarly as the product in Example A-1, the product can be advantageously used as an effective component in prophylactic or therapeutic agents for diseases, as well as a material for preparing HuIFN-gamma derivatives.

### Example B-1

### Liquid medicine

A liquid medicine was obtained by dissolving in physiological saline 50 units/ml of a high-purity HuIFN-gamma prepared by the method in Example A-1, and the mixture was sterilely filtered, and distributed in a 50 ml of sterilized plastic vial, followed by sealing the vial.

The product is suitably prepared into a nebula, a collyrium, a collunarium, or a collutorium for prophylactic or therapeutic agents for viral diseases such as epidemic conjunctivitis and influenza.

### Example B-2

### Injection

A solid injection was obtained by dissolving in physiological saline 100,000 units/ml of a high-purity HuIFN-gamma prepared by the method in Example A-1, and the mixture was sterilely filtered. Two milliliter aliquots of the filtrate was distributed in sterilized glass vials, and lyophilized, followed by sealing the glass vials.

Similarly as the product in Example B-1, the product can be advantageously used in prophylactic or therapeutic agents for viral diseases.

The product is suitable for prophylactic or therapeutic agents for malignant tumors such as breast cancer, lung carcinoma, liver carcinoma and leukemia; and immunopathies such as atopic allergy, pernicious anemia, articular rheumatism and systemic lupus erythematosus.

Furthermore, the product is advantageously used to enhance the antioncotic activity of chemotherapeutics such as melphalan, methotrexate and doxorubicin.

### Example B-3

### Injection

Physiological saline was added with 250,000 units/ml of a high-purity HuIFN-gamma prepared by the method in Example A-2, and in accordance with the method in Example B-2, the mixture was distributed in glass vials, lyophilized and sealed to obtain a solid injection.

Similarly as the product in Example B-2, the product can be advantageously used in prophylactic or therapeutic agents for viral diseases and malignant tumors.

### Example B-4

### Injection

Physiological saline was added with 1,000 units/ml of a high-purity HuIFN-gamma prepared by the method in Example A-3, 100,000 units/ml of lymphoblastoid IFN-alpha and 100,000 units/ml of lymphoblastoid TNF, and the mixture was sterilely filtered, and lyophilized similarly as in Example B-2 to obtain a solid injection.

The product is suitable for prophylactic or therapeutic agents for viral diseases.

The product is also suitable for prophylactic or therapeutic agents for malignant tumors such as breast cancer, lung carcinoma, liver carcinoma, stomach cancer and leukemia; and immunopathies such as atopic allergy, collagenasis, articular rheumatism and systemic lupus erythematosus.

The product can be advantageously used to enhance the antioncotic activity of chemotherapeutics such as tegafur, mitomycin C and vincristine sulfate.

### Example B-5

### Tablet

In the preparation of conventional type of tablet using starch and maltose as a vehicle, a high-purity HuIFN-gamma prepared by the method in Example A-1, and a natural lymphoblastoid TNF were incorporated into a tablet in respective amount of 10,000 units per tablet (200 mg). The obtained tablet was then coated with methylcellulose phthalate to obtain an enteric-coated tablet.

The product is advantageously usable for prophylactic or therapeutic agents for viral diseases such as those in small and large intestines, as well as those for malignant tumors such as colon carcinoma, renal carcinoma and liver carcinoma, and immunopathies such as atopic allergy, pernicious anemia, articular rheumatism and systemic lupus erythematosus.

The product can be advantageously used to enhance the antioncotic activity of chemotherapeutics such as doxorubicin, fluorouracil and mitomycin C.

### Effect of the invention

As detailed in the above, an HuIFN-gamma with a revealed structure, which is essentially derived from a human cell, is advantageously used in prevention or treatment of human diseases.

No detailed structure of an HuIFN-gamma derived from a human cell has hitherto been known, and whether such HuIFN-gamma and the CHO-cell derived HuIFN-gamma are identical or not has not hitherto been clear.

The present invention revealed a structure of a natural HuIFN-gamma, accomplished the process for preparing the same, and obtained prophylactic or therapeutic agents which cause no side effect.

Furthermore, the present invention has a significant effect on the art because the disclosure of the structure of the natural HuIFN-gamma varies the process for preparing HuIFN-gamma on a mass production, and promotes the process for preparing HuIFN-gamma derivatives, as well as promoting the development of novel pharmaceuticals containing substances which have a similar structure to that of the HuIFN-gamma. Therefore, the present invention is significant in the art of pharmaceuticals.

While there has been described what is at present considered to be the preferred embodiments of the invention, it will be understood that various modifications may be made therein, and it is intended to cover in the appended claims all such modifications as fall within the true spirits and scope of the invention.

## Claims

1. A human interferon-gamma which consists essentially of a polypeptide chain having a sequence as shown in any one of the following Formulae:- 
Formula I:
1                                                    10
pyroGlu Asp Pro Tyr Val Lys Glu Ala Glu Asn Leu Lys Lys Tyr Phe
                        20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                        50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                        80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                        110                                                   120
Gln Arg Lys Ala Ile His Glu Leu Ile Gln Val Met Ala Glu Leu
                                                    130
Ser Pro Ala Ala Lys Thr Gly Lys Arg Lys Arg Ser Gln Met Leu

Phe Agr Gly ;

Formula II:

1                                                    10
pyroGlu Asp Pro Tyr Val Lys Glu Ala Glu Asn Leu Lys Lys Tyr Phe
                        20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                        50                                                    60
Gln Ser Gln Ile Val Ser Phe Tyr Phe Lys Leu Phe Lys Asn Phe
 
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys Arg ;

Formula III:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met

50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys;

Formula IV:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
 
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly ;or

Formula V:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr ;
 wherein either or both asparagines which are located at the twenty-fifth and ninety-seventh positions with respect to the NH₂ terminus of the polypeptide chain is coupled to a carbohydrate chain having a structure as shown in any one of the following Formulae:-

Formula VI:
NeuAcα(2→6)Galβ(1→4)GlcNAcβ(1→2)Manα1                    Fucα1
                                                    ↓                              ↓
                                                    6                              6
                                                    Manβ(1→4)GlcNAcβ(1→4)GlcNAc→ ;
                                                    3
                                                    ↑
NeuAcα(2→6)Galβ(1→4)GlcNAcβ(1→2)Manα1

Formula VIII:

NeuAcα(2→3)Galβ(1→4)GlcNAcβ(1→2)Manα 1                    Fucα 1
                                                    ↓                              ↓
                                                    6                              6
                                                    Manβ(1→4)GlcNAcβ(1→4)GlcNAc→ ;
                                                    3
                                                    ↑
NeuAcα(2→6)Galβ(1→4)GlcNAcβ(1→2)Manα 1

Formula X:

                Galβ(1→4)GlcNAcβ(1→2)Manα 1                    Fucα 1
                                                    ↓                              ↓
                                                    6                              6
                                                    Manβ(1→4)GlcNAcβ(1→4)GlcNAc→
                                                    3
                                                    ↑
NeuAcα(2→6)Galβ(1→4)GlcNAcβ(1→2)Manα 1
                                                                                   ;or

Formula XI:

NeuAcα(2→3)Galβ(1→4)GlcNAcβ(1→2)Manα 1                    Fucα 1
                                                    ↓                              ↓
                                                    6                              6
                                                    Manβ(1→4)GlcNAcβ(1→4)GlcNAc→
                                                    3
                                                    ↑
                Galβ(1→4)GlcNAcβ(1→2)Manα 1
                              3
                              ↑
                          Fucα 1          .

2. A human interferon-gamma as claimed in claim 1, wherein said human interferon-gamma has a molecular weight of about 20,000 or about 24,000 daltons on SDS-polyacrylamide gel electrophoresis.

3. A human interferon-gamma as claimed in claim 1 or claim 2, wherein said human interferon-gamma is obtainable by:
exposing a human cell capable of producing said human interferon-gamma to the action of an inducer to allow said human cell to produce said human interferon-gamma; and
recovering said human interferon-gamma.

4. A human interferon-gamma as claimed in claim 3, wherein said human cell is one of a group of proliferated cells obtainable by:
implanting a first human cell capable of producing said human interferon-gamma in a non-human warm-blooded animal; and
allowing said human cell to proliferate while allowing it to receive the body fluid from the non-human warm-blooded animal thereby to form the group of proliferated cells.

5. A human interferon-gamma as claimed in claim 3, wherein said human cell is a member selected from the group consisting of BALL-1 cell (JCRB 0071), TALL-1 cell (JCRB 0086), MOLT-3 cell (ATCC CRL 1552), HBL-38 cell, HL-60 cell (ATCC CCL 240), KG-1 cell (ATCC CCL 246), ML-1 cell and leukocyte cell.

6. A human interferon-gamma as claimed in any one of the preceding claims, wherein said inducer is a member selected from the group consisting of phytohemagglutinin, concanavalin A, pokeweed mitogen, lipopolysaccharide, lipid A, endotoxin, polysaccharide, bacterium, antigen and mixtures thereof.

7. A process for preparing a human interferon-gamma, as defined in claim 1 or claim 2, the process comprising:
exposing a human cell capable of producing said human interferon-gamma to the action of an inducer to allow said human cell to produce said human interferon-gamma; and
recovering said human interferon-gamma.

8. A process as claimed in claim 7, wherein said human cell is one of a group of proliferated cells obtainable by:
implanting a first human cell capable of producing said human interferon-gamma in a non-human warm-blooded animal; and
allowing said human cell to proliferate while allowing it to receive the body fluid from the non-human warm-blooded animal thereby to form the group of proliferated cells.

9. A process as claimed in claim 7, wherein said human cell is a member selected from the group consisting of BALL-1 cell (JCRB 0071), TALL-1 cell (JCRB 0086), MOLT-3 cell (ATCC CRL 1552), HBL-38 cell, HL-60 cell (ATCC CCL 240), KG-1 cell (ATCC CCL 246), ML-1 cell and leukocyte cell.

10. A process as claimed in any one of claims 7, 8 or 9, wherein said inducer is a member selected from the group consisting of phytohemagglutinin, concanavalin A, pokeweed mitogen, lipopolysaccharide, lipid A, endotoxin, polysaccharide, bacterium, antigen and mixtures thereof.

11. A prophylactic or therapeutic agent for human interferon-gamma-susceptive diseases, which contains as an effective ingredient a human interferon-gamma as defined in any one of claims 1 to 6.

12. An agent as claimed in claim 11, wherein said agent contains an additional lymphokine.

13. An agent as claimed in claim 12, wherein said lymphokine is a member selected from the group consisting of interferon-alpha, interferon-beta, tumor necrosis factor, lymphotoxin, interleukin 2, B-cell differentiating factor and mixtures thereof.

14. An agent as claimed in claim 11, wherein said agent is an antiviral agent, antioncotic, enhancer for antioncotic chemotherapeutics, depressant for metastasis of malignant tumors, suppressant for palindromia, immunoregulator, or a therapeutic agent for immunopathies.

15. An agent as claimed in any one of claims 11 to 14, wherein said agent contains said interferon-gamma in the range of 1-10,000,000 units/g.

## Patentansprüche

1. Menschliches Gamma-Interferon, welches im wesentlichen aus einer Polypeptidkette besteht, die eine Sequenz besitzt, wie sie in einer der folgenden Formeln gezeigt ist: 
Formel I:
1                                                    10
pyroGlu Asp Pro Tyr Val Lys Glu Ala Glu Asn Leu Lys Lys Tyr Phe
                    20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                                    60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                    80                                                    90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                    110                                                   120
Gln Arg Lys Ala Ile His Glu Leu Ile Gln Val Met Ala Glu Leu
                                                    130
Ser Pro Ala Ala Lys Thr Gly Lys Arg Lys Arg Ser Gln Met Leu

Phe Agr Gly ;

Formel II:
1                                                    10
pyroGlu Asp Pro Tyr Val Lys Glu Ala Glu Asn Leu Lys Lys Tyr Phe
                    20                                                    30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                                    60
Gln Ser Gln Ile Val Ser Phe Tyr Phe Lys Leu Phe Lys Asn Phe
 
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys Arg ;
Formel III:
1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys;

Formel IV:
1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
 
50                                                    60
Gln Ser Gln Ile Val Ser Phe Tyr Phe Lys Leu Phe Lys Asn Phe
                                        70
Lys Asp Asp Gln Ser Ile Gln Lys Ser Val Glu Thr Ile Lys Glu
                    80                                      90
Asp Met Asn Val Lys Phe Phe Asn Ser Asn Lys Lys Lys Arg Asp
                                        100
Asp Phe Glu Lys Leu Thr Asn Tyr Ser Val Thr Asp Leu Asn Val
                    110                                     120
Gln Arg Lys Ala Ile His Glu Leu Ile Gln Val Met Ala Glu Leu

Ser Pro Ala Ala Lys Thr Gly ; oder
Formel V:
1                                       10
pyroGlu Asp Pro Tyr Val Lys Glu Ala Glu Asn Leu Lys Lys Tyr Phe
                    20                                      30
Asn Ala Gly His Ser Asp Val Ala Asp Asn Gly Thr Leu Phe Leu
                                        40
Gly Ile Leu Lys Asn Trp Lys Glu Glu Ser Asp Arg Lys Ile Met
                    50                                      60
Gln Ser Gln Ile Val Ser Phe Tyr Phe Lys Leu Phe Lys Asn Phe
                                        70
Lys Asp Asp Gln Ser Ile Gln Lys Ser Val Glu Thr Ile Lys Glu
                    80                                      90
Asp Met Asn Val Lys Phe Phe Asn Ser Asn Lys Lys Lys Arg Asp
                                        100
Asp Phe Glu Lys Leu Thr Asn Tyr Ser Val Thr Asp Leu Asn Val
                    110                                     120
Gln Arg Lys Ala Ile His Glu Leu Ile Gln Val Met Ala Glu Leu

Ser Pro Ala Ala Lys Thr ;
 wobei ein oder beide Asparagine, welche an der fünfundzwanzigsten und an der neunundsiebzigsten Position hinsichtlich des NH₂-Terminus der Polypeptidkette angeordnet sind, an eine Kohlenhydratkette gekoppelt sind, welche eine Struktur aufweist, wie sie in einer der folgenden Formeln gezeigt ist:

Formel VI:

Fucα 1
↓
6
NeuAcα (2→6) Gal β (1→4) GlcNAc β (1→2) Man α 1
↓
6
Man β (1→4) GlcNAc β (1→4) GlcNAc→ ;
3
↑
NeuAcα (2→6) Gal β (1→4) GlcNAc β (1→2) Man α 1

Formel VIII:

Fucα 1
↓
6
NeuAcα (2→3) Gal β (1→4) GlcNAc β (1→2) Man α 1
↓
6
Man β (1→4) GlcNAc β (1→4) GlcNAc→ ;
3
↑
NeuAcα (2→6) Gal β (1→4) GlcNAc β (1→2) Man α 1

Formel X:

Fucα 1
↓
6
Gal β (1→4) GlcNAc β (1→2) Man α 1
↓
6
Man β (1→4) GlcNAc β (1→4) GlcNAc→
3
↑
NeuAcα (2→6) Gal β (1→4) GlcNAc β (1→2) Man α 1

oder

Formel XI:

Fucα 1
↓
6
NeuAcα (2→3) Gal β (1→4) GlcNAc β (1→2) Man α 1
↓
6
Man β (1→4) GlcNAc β (1→4) GlcNAc→
3
↑
Gal β (1→4) GlcNAc β (1→2) Man α 1
3
↑
Fucα 1

2. Menschliches Gamma-Interferon, wie in Anspruch 1 beansprucht, wobei das menschliche Gamma-Interferon ein Molekulargewicht von etwa 20.000 oder etwa 24.000 Dalton bei SDS-Polyacrylamidgelelektrophorese besitzt.

3. Menschliches Gamma-Interferon, wie in Anspruch 1 oder 2 beansprucht, wobei das menschliche Gamma-Interferon erhältlich ist durch:
Exponieren einer menschlichen Zelle, die das menschliche Gamma-Interferon produzieren kann, der Wirkung eines Induktors, um die menschliche Zelle das menschliche Gamma-Interferon produzieren zu lassen; und
Gewinnen des menschlichen Gamma-Interferons.

4. Menschliches Gamma-Interferon, wie in Anspruch 3 beansprucht, wobei die menschliche Zelle eine aus einer Gruppe von proliferierten Zellen ist, die erhältlich sind durch:
Implantieren einer ersten menschlichen Zelle, die das menschliche Gamma-Interferon in einem nicht-menschlichen Warmblütler produzieren kann; und
Proliferierenlassen der menschlichen Zelle, während zugelassen wird, daß sie die Körperflüssigkeit aus dem nicht-menschlichen Warmblütler erhält, wodurch die Gruppe der proliferierten Zellen gebildet wird.

5. Menschliches Gamma-Interferon, wie in Anspruch 3 beansprucht, wobei die menschliche Zelle ein Glied, ausgewählt aus der Gruppe von BALL-1-Zelle (JCRB 0071), TALL-1-Zelle (JCRB 0086), MOLT-3-Zelle (ATCC CRL 1552), HBL-38-Zelle, HL-60-Zelle (ATCC CCL 240), KG-1-Zelle (ATCC CCL 246), ML-1-Zelle und Leukozytenzelle, ist.

6. Menschlisches Gamma-Interferon, wie in einem der vorhergehenden Ansprüche beansprucht, wobei der Induktor ein Glied, ausgewählt aus der Gruppe von Phytohämagglutinin, Concanavalin A, Taschenunkraut-Mitogen, Lipopolysaccharid, Lipid A, Endotoxin, Polysaccharid, Bakterium, Antigen und Gemischen davon, ist.

7. Verfahren zur Herstellung eines menschlichen Gamma-Interferons, wie in Anspruch 1 oder Anspruch 2 definiert, welches Verfahren umfaßt:
Exponieren einer menschlichen Zelle die das menschliche Gamma-Interferon produzieren kann, der Wirkung eines Induktors, um die menschliche Zelle das menschliche Gamma-Interferon produzieren zu lassen; und
Gewinnen des menschlichen Gamma-Interferons.

8. Verfahren, wie in Anspruch 7 beansprucht, wobei die menschliche Zelle eine aus einer Gruppe von proliferierten Zellen ist, die erhältlich sind durch:
Implantieren einer ersten menschlichen Zelle, die das menschliche Gamma-Interferon in einem nicht-menschlichen Warmblütler produzieren kann; und
Proliferierenlassen der menschlichen Zelle, während zugelassen wird, daß sie die Körperflüssigkeit aus dem nicht-menschlichen Warmblütler erhält, um dadurch die Gruppe der proliferierten Zellen zu bilden.

9. Verfahren, wie in Anspruch 7 beansprucht, wobei die menschliche Zelle ein Glied, ausgewählt aus der Gruppe von BALL-1-Zelle (JCRB 0071), TALL-1-Zelle (JCRB 0086), MOLT-3-Zelle (ATCC CRL 1552), HBL-38-Zelle, HL-60-Zelle (ATCC CCL 240), KG-1-Zelle (ATCC CCL 246), ML-1-Zelle und Leukozytenzelle, ist.

10. Verfahren, wie in einem der Ansprüche 7, 8 oder 9 beansprucht, wobei der Induktor ein Glied, ausgewählt aus der Gruppe von Phytohämagglutinin, Concanavalin A, Taschenunkraut-Mitogen, Lipopolysaccharid, Lipid A, Endotoxin, Polysaccharid, Bakterium, Antigen und Gemischen davon, ist.

11. Prophylaktisches und therapeutisches Mittel für Krankheiten, die auf menschliches Gamma-Interferon ansprechen, welches Mittel als einen wirksamen Bestandteil ein menschliches Gamma-Interferon, wie es in einem der Ansprüche 1 bis 6 definiert ist, enthält.

12. Mittel, wie in Anspruch 11 beansprucht, wobei das Mittel ein zusätzliches Lymphokin enthält.

13. Mittel, wie in Anspruch 12 beansprucht, wobei das Lymphokin ein Glied, ausgewählt aus der Gruppe von Alpha-Interferon, Beta-Interferon, Tumornekrosefaktor, Lymphotoxin, Interleukin 2, B-Zellen-differenzierender Faktor und Gemischen davon, ist.

14. Mittel, wie in Anspruch 11 beansprucht, wobei das Mittel ein Antivirusmittel, ein Antionkotikum, ein Verstärker für antionkotische Chemotherapeutika, ein Unterdrückungsmittel für die Metastase bösartiger Tumore, ein Unterdrückungsmittel für Palindromie, ein Immunregulator oder ein therapeutisches Mittel für Immunopathien ist.

15. Mittel, wie in einem der Ansprüche 11 bis 14 beansprucht, wobei das Mittel das Gamma-Interferon im Bereich von 1 - 10.000.000 Einheiten/g enthält.

## Revendications

1. Gamma-interféron humain qui est constitué essentiellement d'une chaîne polypeptidique ayant une séquence comme celle représentée par une quelconque des formules suivantes: 
Formule I:

1                                                    10
pyroGlu Asp Pro Tyr Val Lys Glu Ala Glu Asn Leu Lys Lys Tyr Phe
                    20                                          30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                          60
Gln Ser Gln Ile Val Ser Phe Tyr Phe Lys Leu Phe Lys Asn Phe
                                                    70
Lys Asp Asp Gln Ser Ile Gln Lys Ser Val Glu Thr Ile Lys Glu
                    80                                          90
Asp Met Asn Val Lys Phe Phe Asn Ser Asn Lys Lys Lys Arg Asp
                                                    100
Asp Phe Glu Lys Leu Thr Asn Tyr Ser Val Thr Asp Leu Asn Val
                    110                                         120
Gln Arg Lys Ala Ile His Glu Leu Ile Gln Val Met Ala Glu Leu
                                                    130
Ser Pro Ala Ala Lys Thr Gly Lys Arg Lys Arg Ser Gln Met Leu

Phe Agr Gly ;

Formule II:

1                                                    10
pyroGlu Asp Pro Tyr Val Lys Glu Ala Glu Asn Leu Lys Lys Tyr Phe
                    20                                          30
Asn Ala Gly His Ser Asp Val Ala Asp Asn Gly Thr Leu Phe Leu
                                                    40
Gly Ile Leu Lys Asn Trp Lys Glu Glu Ser Asp Arg Lys Ile Met
                    50                                          60
Gln Ser Gln Ile Val Ser Phe Tyr Phe Lys Leu Phe Lys Asn Phe
 
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys Arg ;

Formule III:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
50 50
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu

Ser Pro Ala Ala Lys Thr Gly Lys;

Formule IV:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
 
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu
Ser Pro Ala Ala Lys Thr Gly ;or

Formule V:

1 10
pyroGlu Asp Pro Tyr Val Lys Glu Ala Glu Asn Leu Lys Lys Tyr Phe
20 30
Asn Ala Gly His Ser Asp Val Ala Asp Asn Gly Thr Leu Phe Leu
40
Gly Ile Leu Lys Asn Trp Lys Glu Glu Ser Asp Arg Lys Ile Met
50 60
Gln Ser Gln Ile Val Ser Phe Tyr Phe Lys Leu Phe Lys Asn Phe
70
Lys Asp Asp Gln Ser Ile Gln Lys Ser Val Glu Thr Ile Lys Glu
80 90
Asp Met Asn Val Lys Phe Phe Asn Ser Asn Lys Lys Lys Arg Asp
100
Asp Phe Glu Lys Leu Thr Asn Tyr Ser Val Thr Asp Leu Asn Val
110 120
Gln Arg Lys Ala Ile His Glu Leu Ile Gln Val Met Ala Glu Leu
Ser Pro Ala Ala Lys Thr ;
 où l'une des deux ou les deux asparagines qui sont placées aux trente-cinquième et quatre-vingt-dix-septième positions par rapport à l'extrémité NH₂-terminale de la chaîne polypeptidique est couplée à une chaîne d'hydrate de carbone ayant une structure comme celle représentée par une quelconque des formules suivantes:

Formule VI:

NeuAcα(2→6)Galβ(1→4)GlcNAcβ(1→2)Manα 1                                    Fucα 1
                                                                                                    ↓                                                  ↓
                                                                                                    6                                                  6
                                                                                                    Manβ(1→4)GlcNAcβ(1→4)GlcNAc→ ;
                                                                                                    3
                                                                                                    ↑
NeuAcα(2→6)Galβ(1→4)GlcNAcβ(1→2)Manα 1

Formule VIII:

NeuAcα(2→3)Galβ(1→4)GlcNAcβ(1→2)Manα 1                                    Fucα 1
                                                                                                    ↓                                                  ↓
                                                                                                    6                                                  6
                                                                                                    Manβ(1→4)GlcNAcβ(1→4)GlcNAc→ ;
                                                                                                    3
                                                                                                    ↑
NeuAcα(2→6)Galβ(1→4)GlcNAcβ(1→2)Manα 1

Formule X:

                    Galβ(1→4)GlcNAcβ(1→2)Manα 1                                    Fucα 1
                                                                                                    ↓                                                  ↓ :
                                                                                                    6                                                  6
                                                                                                    Manβ(1→4)GlcNAcβ(1→4)GlcNAc→
                                                                                                    3
                                                                                                    ↑
NeuAcα(2→6)Galβ(1→4)GlcNAcβ(1→2)Manα 1

; ou

Formule XI:

NeuAcα(2→3)Galβ(1→4)GlcNAcβ(1→2)Manα 1                    Fucα 1
                                      ↓                          ↓
                                      6                          6
                                     Manβ(1→4)GlcNAcβ(1→4)GlcNAc→
                                      3
                                      ↑
              Galβ(1→4)GlcNAcβ(1→2)Manα 1
                       3
                       ↑
                    Fucα 1        .

2. Gamma-interféron humain selon la revendication 1,où ledit gamma-interféron humain a un poids moléculaire d'environ 20 000 ou environ 24 000 daltons sur électrophorèse sur gel de dodécyl sulfate de sodium et de polyacrylamide.

3. Gamma-interféron humain selon la revendication 1 ou la revendication 2, où ledit gamma-interféron humain peut être obtenu par:
exposition d'une cellule humaine capable de produire ledit gamma-interféron humain à l'action d'un inducteur pour permettre à ladite cellule humaine de produire ledit gamma-interféron humain; et
collecte dudit gamma-interféron humain.

4. Gamma-interféron humain selon la revendication 3, où ladite cellule humaine provient d'un groupe de cellules ayant
proliféré qui peut être obtenue par
implantation d'une première cellule humaine capable de produire ledit gamma-interféron humain dans un animal à sang chaud autre que l'homme; et
le fait de laisser ladite cellule humaine proliférer tout en la laissant recevoir le fluide corporel provenant de l'animal à sang chaud autre que l'homme pour former le groupe de cellules ayant proliféré.

5. Gamma-interféron humain selon la revendication 3, où ladite cellule humaine est un élément choisi dans le groupe constitué de cellule BALL-1 (JCRB 0071), de cellule TALL-1 (JCRB 0086), de cellule MOLT-3 (ATCC CRL 1552), de cellule HBL-38, de cellule HL-60 (ATCC CCL 240), de cellule KG-1 (ATCC CCL 246), de cellule ML-1 et cellule leucocyte.

6. Gamma-interféron humain selon une quelconque des revendications précédentes, où ledit inducteur est un élément choisi dans le groupe constitué de phytohémagglutinine, de concanavaline A, de mitogène du Pokeweed, de lipopolysaccharide, de lipide A, d'endotoxine, de polysaccharide, de bactérie, d'antigène et de leurs mélanges.

7. Procédé de préparation d'un gamma-interféron humain, tel que défini dans la revendication 1 ou la revendication 2, le procédé comprenant:
l'exposition d'une cellule humaine capable de produire ledit gamma-interféron humain à l'action d'un inducteur pour permettre à ladite cellule humaine de produire ledit gamma-interféron humain; et
la collecte dudit gamma-interféron humain.

8. Procédé selon la revendication 7, où ladite cellule humaine est l'une d'un groupe de cellules ayant proliféré qui peut être obtenue par:
implantation d'une première cellule humaine capable de produire ledit gamma-interféron humain dans un animal à sang chaud autre que l'homme; et
le fait de laisser proliférer ladite cellule humaine tout en la laissant recevoir le fluide corporel provenant de l'animal à sang chaud autre que l'homme pour former ainsi le groupe de cellules ayant proliféré.

9. Procédé selon la revendication 7, où ladite cellule humaine est un élément choisi dans le groupe constitué de cellule BALL-1 (JCRB 0071), de cellule TALL-1 (JCRB 0086), de cellule MOLT-3 (ATCC CRL 1552), de cellule HBL-38, de cellule HL-60 (ATCC CCL 240), de cellule KG-1 (ATCC CCL 246), de cellule ML-1 et cellule leucocyte.

10. Procédé selon une quelconque des revendications 7, 8 ou 9, où ledit inducteur est un élément choisi dans le groupe constitué de phytohémagglutinine, de concanavaline A, de mitogène de Pokeweed, de lipopolysaccharide, de lipide A, d'endotoxine, de polysaccharide, de bactérie, d'antigène et de leurs mélanges.

11. Agent prophylactique ou thérapeutique destiné aux maladies sensibles au gamma-interféron humain, qui contient comme ingrédient efficace un gamma-interféron humain selon une quelconque des revendications 1 à 6.

12. Agent selon la revendication 11, où ledit agent contient un lymphokyne supplémentaire.

13. Agent selon la revendication 12, où ledit lymphokyne est un élément choisi dans le groupe constitué d'alpha-interféron, de béta-interféron, de facteur de nécrose tumorale de lymphotoxine, d'interleukine 2, de facteur de différenciation des lymphocytes B et de leurs mélanges.

14. Agent selon la revendication 11, où ledit agent est un agent antiviral, un antioncotique, un activateur de chimiothérapie antioncotique, un médicament abaissant l'activité métastasique des tumeurs malignes, un médicament antipalindromique, un immunorégulateur, ou un agent thérapeutique destiné aux immunopathies.

15. Agent selon une quelconque des revendications 11 à 14, ou ledit agent contient ledit gamma-interféron dans la plage de 1 à 10 000 000 unités/g.
